# FASCICULE DE BREVET EUROPEEN

(11) **EP 0 671 160 B1**
(45) Date de publication et mention de la délivrance du brevet: **07.08.1996**
(21) Numéro de dépôt: 95400343.0
(22) Date de dépôt: 17.02.1995
(51) Int. Cl.: A61K 7/42, A61K 7/48

(54) **Utilisation des acides sulfoniques comme agents antivieillissement dans une composition cosmétique ou dermatologique**
Verwendung von Sulfonsäuren als Mittel gegen Hautalterung in einer kosmetischen und/oder dermatologischen Zusammensetzung
Use of sulphonic acids as antiageing agents in cosmetic and/or dermatological compositions

(30) Priorité: 08.03.1994 FR 9402657
(43) Date de publication de la demande: 13.09.1995
(73) Titulaire: L'OREAL, F-75008 Paris (FR)
(72) Inventeur: Boelle, Jean-Paul, F-92190 Meudon (FR); Laugier, Jean-Pierre, F-92160 Antony (FR); Forestier, Serge, F-77410 Claye Souilly (FR)
(74) Mandataire: Lhoste, Catherine

(56) Documents cités:
- EP-A- 0 457 687
- EP-A- 0 531 192
- FR-A- 2 645 148
- GB-A- 2 185 019

## Description

La présente invention a pour objet une nouvelle composition cosmétique et/ou dermatologique destinée à lutter contre le vieillissement cutané intrinsèque.

Les signes du vieillissement cutané résultant des effets sur la peau des facteurs intrinsèques ou extrinsèques, se définissent par l'apparition de rides et ridules, par le jaunissement de la peau qui développe un aspect parcheminé accompagné de l'apparition de taches pigmentaires, par la modification de l'épaisseur de la peau, entraînant généralement un épaississement du *stratum corneum* et de l'épiderme et un amincissement du derme, par la désorganisation des fibres d'élastine et de collagène d'où une perte d'élasticité, de souplesse et de fermeté et par l'apparition de télangiectasies.

Certains de ces signes sont plus particulièrement liés au vieillissement intrinsèque ou physiologique, c'est-à-dire au vieillissement « normal » lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

L'invention s'intéresse uniquement au vieillissement intrinsèque ou physiologique. Les changements de la peau qui se produisent du fait du vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur diverses compositions cosmétiques destinées à lutter contre le vieillissement cutané.

L'acide rétinoïque et ses dérivés ont été décrits comme des agents antivieillissement dans des compositions cosmétiques notamment dans le brevet US 4.603.146.

On connaît également dans cette même application les α-hydroxyacides comme l'acide lactique, l'acide glycolique ou encore l'acide citrique qui ont été décrits dans de nombreux brevets et publications (voir par exemple la demande EP-A- 413 528) et introduits dans de nombreuses compositions cosmétiques proposées sur le marché.

Ont été également proposés les ortho-hydroxyacides aromatiques comme l'acide salicylique (voir par exemple les demandes WO 93/10756 et WO 93/10755).

Tous ces composés ont une action contre le vieillissement de la peau, consistant en une desquamation, c'est-à-dire l'élimination des cellules mortes situées à la surface du *stratum corneum*. Cette desquamation est aussi appelée propriété kératolytique. Mais ces composés présentent également des effets secondaires, qui consistent en des picotements et des rougeurs désagréables pour l'utilisateur.

On constate donc que subsiste le besoin d'agents antivieillissement ayant une action au moins aussi efficace que les composés de l'art antérieur, mais ne présentant pas leurs inconvénients, et en particulier d'agents agissant sur le vieillissement lié à l'âge.

C'est ce qu'apporte la présente invention.

La demanderesse a découvert que pouvaient être utilisés dans une composition cosmétique en tant qu'agents contre le vieillissement intrinsèque ou physiologique de la peau, les composés présentant au moins une fonction acide sulfonique au moins partiellement non neutralisée.

La demanderesse a constaté, sans que cela soit complètement expliqué, que ces composés ont une action antivieillissement au moins aussi efficace que les composés de l'art antérieur qui sont tous des acides carboxyliques et que cette action est plus douce dans la mesure où aucune irritation n'est ressentie et aucune rougeur n'est observée lors de l'application sur la peau d'une composition cosmétique ou dermatologique les contenant.

Certains de ces acides sulfoniques ont été précédemment utilisés dans des compositions antisolaires pour protéger la peau du rayonnement ultraviolet, c'est à dire pour protéger la peau des érythèmes et des brûlures provoqués par les radiations lumineuses de longueur d'onde comprise entre 280 et 400 nm, plus particulièrement entre 280 et 360 nm. De telles compositions sont notamment décrites dans les documents suivants : US 4.585.597, FR 2.236.515, 2.282.426, 2.645.148, 2.430.938 et 2.592.380. La protection solaire permet de protéger la peau du photovieillissement, mais elle ne permet pas de traiter le vieillissement intrinsèque.

En outre, aucun de ces documents ne suggère le traitement du vieillissement physiologique par ces acides sulfoniques.

Ces acides sulfoniques sont utilisés généralement sous leur forme totalement neutralisée, forme inapte au traitement du vieillissement intrinsèque de la peau.

La présente invention a donc pour objet l'utilisation d'au moins un composé présentant au moins une fonction acide sulfonique au moins partiellement non neutralisée comme agent pour lutter contre le vieillissement intrinsèque dans et pour la fabrication d'une composition cosmétique et/ou dermatologique.

La présente invention a aussi pour objet l'utilisation d'au moins un composé présentant au moins une fonction acide sulfonique au moins partiellement non neutralisée dans et pour la fabrication d'une composition cosmétique et/ou dermatologique pour lutter contre les rides et/ou ridules dues au vieillissement intrinsèque.

La présente invention a encore pour objet l'utilisation d'au moins un composé présentant au moins une fonction acide sulfonique au moins partiellement non neutralisée dans et pour la fabrication d'une composition cosmétique et/ou dermatologique pour éliminer les cellules mortes de la peau.

La présente invention a de plus pour objet un procédé non thérapeutique pour lutter contre le vieillissement cutané intrinsèque, caractérisé en ce qu'il consiste à appliquer sur la peau une composition cosmétique et/ou dermatologique contenant au moins un composé présentant au moins une fonction acide sulfonique au moins partiellement non neutralisée.

La présente invention présente de plus un intérêt tout particulier lorsque l'on met en oeuvre les acides sulfoniques qui sont utilisés comme filtre solaire dans l'art antérieur sous leur forme neutralisée. En effet, dans ce cas là, la peau est également protégée des effets nocifs du rayonnement ultraviolet, ceci grâce à la capacité qu'ont ces acides sulfoniques spécifiques, même sous leur forme acide, de filtrer le rayonnement solaire.

Les acides sulfoniques pouvant être mis en oeuvre dans le cadre de la présente invention peuvent être représentés par la formule générale (a) :

R-SO₃H (a)

dans laquelle R représente un reste hydrocarboné aliphatique ou aromatique.

Selon un mode de réalisation particulier de l'invention, R est choisi parmi le groupe comprenant les radicaux alkyle ayant de 1 à 30 atomes de carbone environ et alcényle ayant de 2 à 30 atomes de carbone environ, linéaires ou ramifiés et éventuellement substitués par un ou plusieurs groupements hydroxyle, alcoxy, acyloxy, aryle, cycloalkyle ou polycycloalkyle.

On peut citer comme exemples de tels composés :
l'acide méthane sulfonique CH₃SO₃H,
l'acide éthane sulfonique CH₃CH₂SO₃H,
l'acide n-butane 1-sulfonique CH₃CH₂CH₂CH₂SO₃H,
l'acide n-dodécane 1-sulfonique CH₃-(CH₂)₁₁-SO₃H,
l'acide n-octadécane 1-sulfonique CH₃-(CH₂)₁₇-SO₃H,
l'acide vinyl sulfonique CH₂=CHSO₃H,
l'acide 2-hydroxyéthane 1-sulfonique HO-CH₂CH₂SO₃H,
l'acide 4-hydrobutane 1-sulfonique HO-(CH₂)₄SO₃H,
l'acide campho-10-sulfonique.

Selon un autre mode de réalisation particulier de l'invention, R est un reste aryle ayant de 6 à 10 atomes de carbone éventuellement substitué par un ou plusieurs groupements hydroxyle, alcoxy, alcényloxy, alkyle, acyloxy, aryle, aryloxy, aroyle, aroyloxy, carboxyle, halogèno ou sulfonyle.

On peut citer comme exemple de tels composés :
l'acide benzène sulfonique : 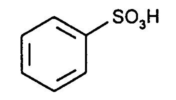
l'acide 4-méthyl benzène sulfonique : 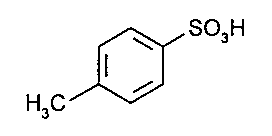
l'acide dodécyl benzène sulfonique (mélange d'isomères) : 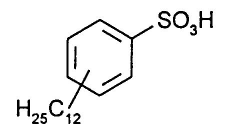
l'acide 5-sulfo salicylique : 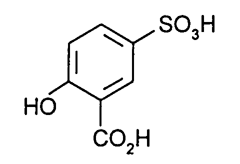
l'acide 5-sulfo isophtalique : 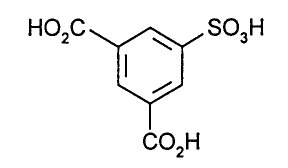
l'acide 4-méthoxy benzène sulfonique : 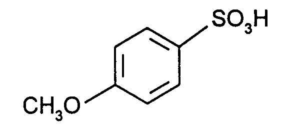
l'acide xylène sulfonique (mélange d'isomères) : 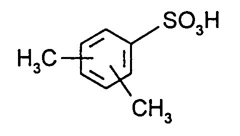
l'acide 3,6-dihydroxynaphtalène 2,7-disulfonique : 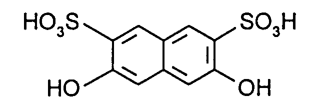
l'acide 1,8-dihydroxynaphtalène 3,6-disulfonique : 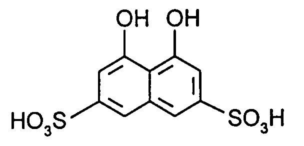
l'acide 4-chloro benzène sulfonique :

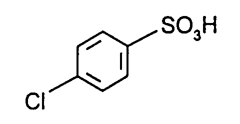

Selon un autre mode de réalisation particulier de l'invention, on met en oeuvre des dérivés sulfoniques ayant de plus la propriété de filtrer le rayonnement UV.

On peut citer comme exemples de tels composés ceux ayant la formule générale (b) suivante : 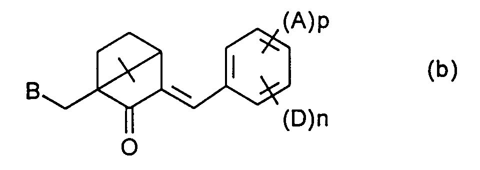
dans laquelle :
B représente H ou SO₃H
0≦p≦1 avec B = SO₃H quand p = 0
0≦n≦4
D représente un ou plusieurs radicaux alkyle, alcényle, alcényloxy ou alcoxy, identiques ou différents quand n ≧ 2, linéaires ou ramifiés contenant de 1 à 18 atomes de carbone environ, un radical halogéno, hydroxyle.

A, de préférence en méta ou en para, représente
soit un radical SO₃H ;
soit un groupement: 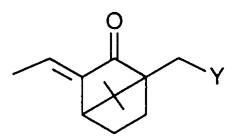
dans lequel Y représente H ou SO₃H ;
soit un groupement: 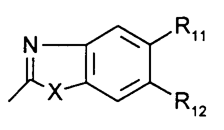
dans lequel :
R₁₁ désigne un atome d'hydrogène, un radical alkyle ou alcoxy, linéaire ou ramifié contenant de 1 à 6 atomes de carbone environ ou le radical SO₃H, R₁₁ étant SO₃H lorsque B = H,
R₁₂ désigne un atome d'hydrogène, un radical alkyle ou alcoxy, linéaire ou ramifié, contenant de 1 à 6 atomes de carbone environ,
et X est un atome d'oxygène, de soufre ou un groupement -NR-, R étant un atome d'hydrogène ou un radical alkyle, linéaire ou ramifié, contenant de 1 à 6 atomes de carbone environ.

On peut citer comme exemples particuliers de composés de formule (b) les dérivés de formules (I), (II), (III) suivantes : 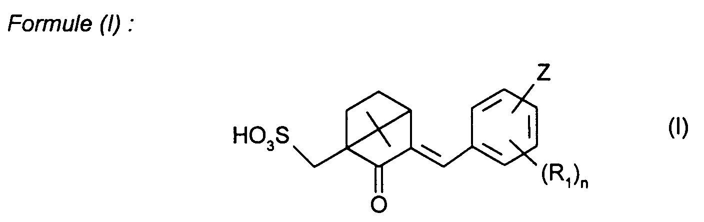
dans laquelle :
- Z, de préférence en position para ou méta, désigne un groupement : 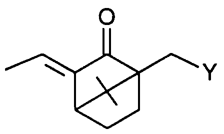 dans lequel Y représente H ou SO₃H
- n est égal à 0 ou est un nombre entier compris entre 1 et 4 (0≦n≦4)
- R₁, représente un ou plusieurs radicaux alkyle ou alkoxy, identiques ou différents, linéaires ou ramifiés, contenant de 1 à 4 atomes de carbone environ.

Un composé de formule I particulièrement préféré est celui correspondant à n = 0 , Z en position para et Y = SO₃H : l'acide benzène 1,4 [di(3-méthylidénecampho-10-sulfonique)]. 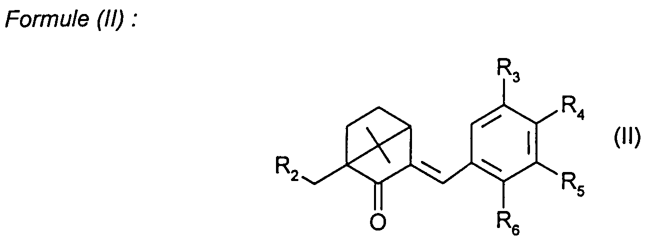
dans laquelle :
- R₂ désigne un atome d'hydrogène ou un radical -SO₃H.
- R₃, R₄, R₅ et R₆, identiques ou différents, représentent un groupement hydroxyle, un radical alkyle ayant de 1 à 4 atomes de carbone environ, linéaire ou ramifié, un radical alcényle ayant de 2 à 4 atomes de carbone environ, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno; de plus un radical R₃ à R₆ seulement peut être un radical SO₃H, au moins un des radicaux R₃ à R₆ désignant le radical -SO₃H quand R₂ est un atome d'hydrogène.

On peut citer comme exemples particuliers les composés suivants de formule II dans laquelle :
- R₄ désigne le radical -SO₃H en position para du benzylidènecamphre et R₂, R₃, R₅ et R₆ désignent chacun un atome d'hydrogène, c'est-à-dire l'acide 4'-sulfo 3-benzylidènecamphre.
- R₃, R₄, R₅ et R₆ désignent chacun un atome d'hydrogène et R₂ désigne un radical SO₃H, c'est-à-dire l'acide 3-benzylidène campho-10-sulfonique.
- R₄ désigne un radical méthyle en position para du benzylidènecamphre, R₅ un radical -SO₃H et R₂, R₃ et R₆ représentent un atome d'hydrogène, c'est-à-dire l'acide 4'-méthyl 3'-sulfo 3-benzylidènecamphre.
- R₄ désigne un atome de chlore en position para du benzylidènecamphre, R₅ un radical -SO₃H et R₂, R₃ et R₆ représentent un atome d'hydrogène, c'est-à-dire l'acide 4'-chloro 3'-sulfo 3-benzylidènecamphre.
- R₄ désigne un radical méthyle en position para du benzylidènecamphre, R₃, R₅ et R₆ désignent un atome d'hydrogène et R₂ désigne un radical -SO₃H, c'est-à-dire l'acide 4'-méthyl 3-benzylidène campho-10-sulfonique.
- R₂ représente un radical SO₃H, R₃ est un radical méthyle, R₄ un atome d'hydrogène, R₅ un radical tertiobutyle, R₆ un radical hydroxyle, c'est-à-dire l'acide 3-(3-t-butyl 2-hydroxy 5-méthyl) benzylidène campho-10-sulfonique.
- R₂ représente un radical SO₃H, R₃ est un radical méthoxy, R₄ un atome d'hydrogène, R₅ un radical tertiobutyle, R₆ un radical hydroxyle, c'est-à-dire l'acide 3-(3-t-butyl 2-hydroxy 5-méthoxy) benzylidène campho-10-sulfonique.
- R₂ représente un radical SO₃H, R₃ et R₅ désignent chacun un radical tertiobutyle, R₄ un radical hydroxyle, R₆ un atome d'hydrogène, c'est-à-dire l'acide 3-(3,5-diterbutyl 4-hydroxy) benzylidène campho-10-sulfonique.
- R₄ représente un radical méthoxy en para, R₅ représente SO₃H, les radicaux R₂, R₃ et R₆ représentent H, c'est-à-dire l'acide 4'-méthoxy 3'-sulfo 3-benzylidènecamphre.
- R₂ désigne un radical -SO₃H, R₃ et R₆ représentent H, R₄ et R₅ formant un radical méthylènedioxy, c'est-à-dire l'acide 3-(4,5-méthylènedioxy) benzylidène campho-10-sulfonique.
- R₂ représente un radical -SO₃H, R₄ un radical méthoxy et les radicaux R₃, R₅ et R₆ représentent H, c'est-à-dire l'acide 3-(4-méthoxy) benzylidène campho-10-sulfonique.
- R₂ représente un radical -SO₃H, R₄ et R₅ sont tous deux un radical méthoxy et les radicaux R₃ et R₆ représentent H, c'est-à-dire l'acide 3-(4,5-diméthoxy) benzylidène campho-10-sulfonique.
- R₂ représente un radical -SO₃H, R₄ est un radical n-butoxy et les radicaux R₃, R₅ et R₆ représentent un atome d'hydrogène, c'est-à-dire l'acide 3-(4-n.butoxy) benzylidène campho-10-sulfonique.
- R₂ représente un radical -SO₃H, R₄ est un radical n-butoxy, R₅ est un radical méthoxy et R₃ et R₆ désignent tous deux un atome d'hydrogène, c'est-à-dire l'acide 3-(4-n.butoxy 5-méthoxy) benzylidène campho-10-sulfonique.

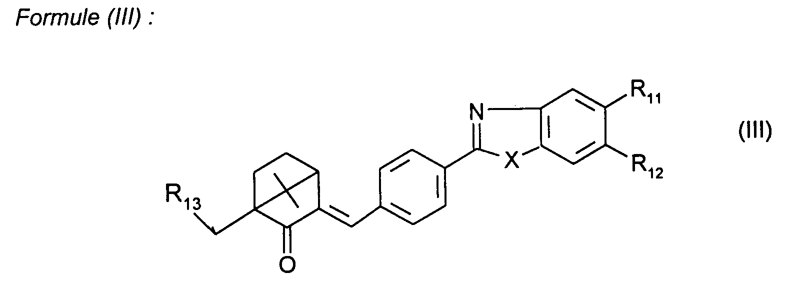

dans laquelle :
- R₁₁ désigne un atome d'hydrogène, un radical alkyle ou alcoxy, linéaire ou ramifié, contenant de 1 à 6 atomes de carbone environ ou un radical -SO₃H,
- R₁₂ désigne un atome d'hydrogène, un radical alkyle ou alcoxy, linéaire ou ramifié, contenant de 1 à 6 atomes de carbone environ,
- R₁₃ désigne un atome d'hydrogène ou un radical -SO₃H,
- l'un au moins des radicaux R₁₁ et R₁₃ désignant un radical -SO₃H,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de formule (III) : le composé dans lequel X désigne un radical -NH-, R₁₁ désigne un radical -SO₃H, R₁₂ et R₁₃ désignent tous deux un atome d'hydrogène, c'est-à-dire l'acide 2-[4-(camphométhylidène) phényl] benzimidazole-5-sulfonique.

Les composés de structures (I), (II), (III) sont décrits dans le brevet US 4.585.597 et les brevets FR 2.236.515, 2.282.426, 2.645.148, 2.430.938, 2.592.380.

On peut citer comme autres exemples de composés ayant également la propriété de filtrer le rayonnement UV les composés de formule générale (c) suivante : 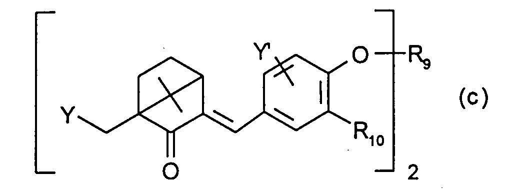
dans laquelle :
- R₉ désigne un radical divalent : -(CH₂)ₘ- ou -CH₂ -CHOH-CH₂- , m étant un nombre entier compris entre 1 et 10 (1 ≦ m ≦ 10),
- R₁₀ désigne un atome d'hydrogène, un radical alcoxy contenant de 1 à 4 atomes de carbone environ,
- Y et Y' désignent un atome d'hydrogène ou un radical -SO₃H, au moins un de ces radicaux Y ou Y' est différent de l'hydrogène.

On peut citer comme exemples particuliers les composés suivants de formule (c) dans laquelle Y représente SO₃H, Y' est H, R10 est H et R9 est -CH₂-CH₂-, c'est à dire l'acide éthylène bis [3(4'-oxy benzylidène) campho-10-sulfonique].

On peut encore citer comme exemples de dérivés sulfoniques ayant de plus la propriété de filtre du rayonnement UV les composés de formule générale (d) suivante : 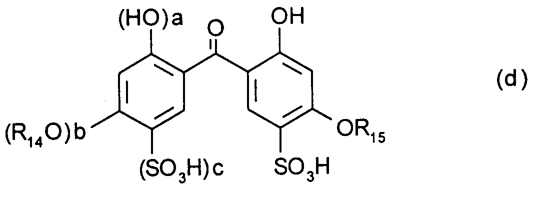
dans laquelle :
- R₁₄ et R₁₅, identiques ou différents, désignent soit un atome d'hydrogène soit un radical alkyle, linéaire ou ramifié, contenant de 1 à 8 atomes de carbone environ.
- a, b et c, identiques ou différents, sont égaux à 0 ou 1.

On peut citer comme exemple particulier de composé de formule (d) : l'acide 2-hydroxy 4-méthoxybenzophénone 5-sulfonique (composé de formule (d) dans laquelle a, b, et c sont égaux à zéro, et R₁₅ désigne un radical méthyle), désigné dans le dictionnaire CTFA par benzophénone-4.

Un autre exemple de dérivés sulfoniques ayant également la propriété de filtrer le rayonnement UV a la formule générale (e) : 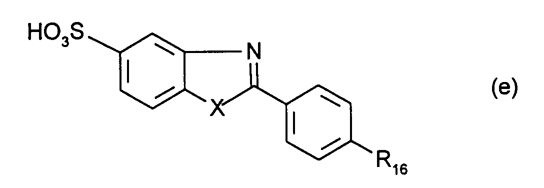
dans laquelle :
- X désigne un atome d'oxygène ou un radical -NH-
- R₁₆ désigne un atome d'hydrogène, un radical alkyle ou alcoxy, linéaire ou ramifié, contenant de 1 à 8 atomes de carbone environ ou un groupement de formule 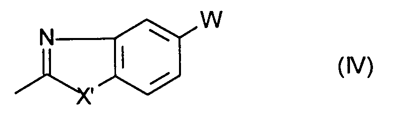 dans laquelle X' représente un atome d'oxygène ou un radical -NH- et W représente H ou SO₃H.

On peut citer comme exemples particuliers les composés suivants de formule (e) dans laquelle:
- X désigne le radical -NH- et R₁₆ désigne un atome d'hydrogène : l'acide 2-phénylbenzimidazole 5-sulfonique, désigné par "2-phenylbenzimidazole 5-sulfonic acid" dans le dictionnaire CTFA.
- X désigne le radical -NH-, R₁₆ désigne le groupement de formule (IV) dans lequel W représente SO₃H et X' désigne le radical -NH- : l'acide benzène 1,4 -di(benzimidazol-2 yl-5-sulfonique).
- X désigne un atome d'oxygène, R₁₆ désigne le groupement de formule (IV) dans lequel W représente SO₃H et X' désigne un atome d'oxygène : l'acide benzène 1,4-di (benzoxazol -2 yl-5-sulfonique).

Les composés de formule (d) et (e) sont des composés connus pouvant être préparés selon des méthodes classiques décrites dans l'art antérieur.

Les acides sulfoniques sont utilisés, selon la présente invention, en quantité de préférence comprise entre 0,1 et 10 % en poids et encore plus préférentiellement entre 0,1 et 5 %.

Le taux de neutralisation ne doit pas être total. Plus celui-ci sera faible, plus l'action antivieillissement proprement dite de la composition sera importante.

La neutralisation est obtenue de façon classique à l'aide d'une base minérale ou organique comme par exemple la soude, la potasse, l'ammoniaque, la monoéthanolamine, la triéthanolamine, l'isopropanolamine etc.

Les compositions cosmétiques selon l'invention peuvent contenir un ou plusieurs filtres solaires complémentaires actifs dans l'UVA et/ou l'UVB, hydrophiles ou lipophiles autres, bien sûr, que les agents hydrophiles acides utilisés dans la présente invention.

Ces filtres complémentaires sont choisis de préférence parmi les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3(4-méthylbenzylidène)camphre, les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxydibenzoylméthane, les dérivés de β,β-diphénylacrylate tels que le α-cyano-β,β-diphénylacrylate de 2-éthylhexyle, (2-cyano, 3,3-diphényl 2-propénoate d'octyle), les dérivés de l'acide p-aminobenzoïque comme par exemple le paradiméthylaminobenzoate d'octyle, l'anthranilate de menthyle, les polymères filtres et silicones filtres décrits dans la demande WO-93-04665.

A titre de filtre complémentaire, on peut également utiliser des substances minérales comme les nanopigments d'oxyde métallique, oxyde de titane, de fer, de zinc, de zirconium, notamment.

Les compositions de l'invention peuvent comprendre en outre les adjuvants cosmétiques classiquement mis en oeuvre comme les corps gras, les solvants organiques, les épaississants ioniques ou non ioniques, les adoucissants, les antioxydants, les opacifiants, les stabilisants, les silicones, les agents anti-mousse, les agents hydratants, les vitamines, les parfums, les conservateurs, les tensioactifs ioniques ou non, les charges, les séquestrants, les colorants ou tout autre ingrédient habituellement utilisé en cosmétique.

Les compositions selon l'invention sont préparées selon les techniques bien connues de l'homme de l'art du domaine.

Les compositions selon l'invention peuvent se présenter sous forme de suspension ou de dispersion dans des solvants ou des corps gras, sous forme de dispersion vésiculaire ou encore sous forme d'émulsion telle qu'une crème ou un lait, sous forme de pommade, de gel, de bâtonnet solide, de mousse aérosol ou de spray.

Les exemples suivants illustrent l'invention sans en limiter aucunement la portée. Les pourcentages sont donnés en poids.

### Exemple 1

On prépare une crème de soin ayant la composition suivante :

| | |
|---|---|
| Span 65 ® (ICI) (Sorbitan tristearate) | 1 % |
| Monostéarate de glycérol | 3 % |
| Myrj 52 ® (ICI) (PEG-40 stearate) | 2 % |
| Alcool cétostéarylique (50-50) | 4 % |
| Perhydrosqualène | 15 % |
| Huile de vaseline | 2 % |
| Glycérol | 3 % |
| Sepigel 305 ® (SEPPIC) (Polyacrylamide/C₁₃-C₁₄ Isoparaffin/Laureth-7) | 1 % |
| EDTA Disodique | 0,05 % |
| Conservateurs | 0,1 % |
| Acide benzène 1,4 [di(3-méthylidènecampho-10-sulfonique)] | 0,7 % |
| Eau déminéralisée | qsp 100 % |
| pH de la formule : 2 | |

### Exemple 2

On prépare une crème de soin ayant la composition suivante :

| | |
|---|---|
| Span 65 ® (ICI) (Sorbitan tristearate) | 1 % |
| Monostéarate de glycérol | 3 % |
| Myrj 52 ® (ICI) (PEG-40 stearate) | 2 % |
| Alcool cétostéarylique (50-50) | 4 % |
| Perhydrosqualène | 15 % |
| Huile de vaseline | 2 % |
| Glycérol | 3 % |
| Sepigel 305 ® (SEPPIC) (Polyacrylamide/C₁₃-C₁₄ Isoparaffin/Laureth-7) | 1 % |
| EDTA Disodique | 0,05 % |
| Conservateurs | 0,1 % |
| Acide benzène 1,4 [di(3-méthylidènecampho-10-sulfonique)] | 0,7 % |
| Eau déminéralisée | qsp 100 % |
| Triéthanolamine | 0,4 % |
| pH de la formule : 4 | |

### Exemple 3

On prépare un gel ayant la composition suivante :

| | |
|---|---|
| Carbopol 934 P ® (GOODRICH) (carbomer) | 1 % |
| Glycérol | 3 % |
| Propylène glycol | 3 % |
| Gomme xanthane | 0,4 % |
| Eusolex 232 ® (MERCK) (Phenylbenzimidazole sulfonic acid) | 1 % |
| Triéthanolamine | 0,1 % |
| Eau déminéralisée | qsp 100 % |
| pH de la formule : 4 | |

### Exemple 4

On prépare un sérum ayant la composition suivante :

| | |
|---|---|
| Miglyol 812 ® (DYNAMIT NOBEL) (caprylic/capric Triglyceride) | 0,5 % |
| EDTA disodique | 0,05 % |
| Parfum / Conservateurs | 0,5 % |
| Carbopol 940 ® (GOODRICH) (carbomer) | 0,3 % |
| Gomme Xanthane | 0,15 % |
| Glycérine | 3 % |
| Alcool cétylique | 0,1 % |
| Diéthanolamine de coprah | 0,1 % |
| Monostéarate de glycérol | 0,3 % |
| Stéarate de PEG 100 | 0,3 % |
| Acide benzène 1,4 [di(3-méthylidènecampho-10 sulfonique)] | 1 % |
| Eau déminéralisée | qsp 100 % |
| pH de la formule : 3,8 | |

### Exemple 5 :

On prépare une crème ayant la composition suivante :

| | |
|---|---|
| Myrj 52(I.C.I.) (PEG-40 stearate) | 2 % |
| Span 65 ® (I.C.I.) (Sorbitan tristearate) | 1 % |
| Monostéarate de glycérol | 2,5 % |
| Huile de vaseline | 2 % |
| Cyclométhicone | 15 % |
| Propylène glycol | 3 % |
| Glycérine | 3 % |
| Alcool cétylique | 3 % |
| Acide campho-10-sulfonique | 1 % |
| Triéthanolamine | 0,3 % |
| Eau déminéralisée | qsp 100 % |

### Exemple 6 :

On prépare une crème ayant la composition suivante :

| | |
|---|---|
| Myrj 52 ® (I.C.I.) (PEG-40 stearate) | 1,5 % |
| Span 65 ® (I.C.I.) (Sorbitan tristearate) | 1 % |
| Monostéarate de glycérol | 2,5 % |
| Huile de vaseline | 3 % |
| Parsol MCX® (Givaudan) (Octyl methoxycinnamate) | 0,5 % |
| Huile de noyaux d'abricot | 13 % |
| Propylène glycol | 3 % |
| Glycérine | 3 % |
| Alcool cétostéarylique | 3 % |
| UVINUL MS 40® (B.A.S.F.) (Benzophenone-4) | 1 % |
| Eau déminéralisée | qsp 100 % |

### Exemple 7 :

On prépare une crème ayant la composition suivante :

| | |
|---|---|
| Myrj 52 ® (I.C.I.) (PEG-40 stearate) | 2 % |
| Span 65 ® (I.C.I.) (Sorbitan tristearate) | 1 % |
| Monostéarate de glycérol | 3 % |
| Alcool cétylique | 2 % |
| Huile de vaseline | 17 % |
| Glycérol | 3 % |
| Sepigel 305 ® (SEPPIC) (Polyacrylamide/C₁₃-C₁₄ Isoparaffin/Laureth-7) | 1 % |
| EDTA disodique | 0,05 % |
| Acide éthane sulfonique | 0,5 % |
| Triéthanolamine | 0,1 % |
| Eau déminéralisée | qsp 100 % |

### Exemple 8 :

On prépare un fluide ayant la composition suivante :

| | |
|---|---|
| Lécithine de soja hydrogénée | 0,5 % |
| Générol 122 E5 ® (HENKEL) (PEG-5 Soya Sterol) | 0,2 % |
| Huile de noyaux d'abricot | 2,5 % |
| EDTA disodique | 0,05 % |
| Sepigel 305 ® (SEPPIC) (Polyacrylamide/C₁₃-C₁₄ Isoparaffin/Laureth-7) | 0,15 % |
| CARBOPOL 980 ® (GOODRICH) (carbomer) | 0,4 % |
| Glycérine | 6 % |
| Eau déminéralisée | qsp 100 % |
| Eusolex 232 ® (MERCK) (Phenylbenzimidazole sulfonic acid) | 0,5 % |
| Acide benzène 1,4[di(méthylidène campho-10-sulfonique)] | 0,5 % |
| Triéthanolamine | 0,2 % |

### Exemple 9 :

On prépare une crème de soin ayant la composition suivante :

| | |
|---|---|
| Myrj 52 ® (I.C.I.) (PEG-40 stearate) | 2 % |
| Span 65 ® (I.C.I.) (Sorbitan tristearate) | 1 % |
| Monostéarate de glycérol | 2 % |
| PARSOL MCX ® (MERCK) (Octyl methoxycinnamate) | 1 % |
| Acide benzène 1,4[di(méthylidène campho-10-sulfonique)] | 0,7 % |
| Propylène glycol | 3 % |
| Glycérol | 3 % |
| Alcool cétylique | 3 % |
| Eau déminéralisée | qsp 100 % |
| Huile de tournesol | 18 % |
| Triéthanolamine | 0,1 % |

### Exemple 10 :

On prépare une lotion tonique ayant la composition suivante :

| | |
|---|---|
| Acide benzène 1,4[di(méthylidène campho 10-sulfonique)] | 0,5 % |
| UVINUL MS 40 ® (BASF) (Benzophenone-4) | 0,5 % |
| Paraoxybenzoate de méthyle | 0,1 % |
| Triéthanolamine | 0,1 % |
| EDTA disodique | 0,05 % |
| PEMULEN TR1® (GOODRICH) (Acrylates/C₁₀-C₃₀ alkyl acrylate crosspolymer) | 0,15 % |
| Glycérine | 3 % |
| Propylèneglycol | 2 % |
| D Panthénol | 0,2 % |
| Eau déminéralisée | qsp 100 % |

### Exemple 11

On prépare une crème de soin ayant la composition suivante :

| | |
|---|---|
| Huile de vaseline | 15,0 % |
| Demsconet 390 ® (Tensia) (cetearyl alcohol /ceteareth-33) | 7,0 % |
| Géléol Copeaux ® (Gattefossé)(Glyceryl stearate) | 2,0 % |
| Lorol C16 ® (Henkel) (cetyl alcohol) | 1,5 % |
| Silbione 70 047 V 300 ® (Rhône-Poulenc) | 1,5 % |
| Acide benzène -1,4[di(3-méthylidènecampho-10-sulfonique)] | 2,0 % |
| Parsol MCX ® (Givaudan Roure) (Octyl methoxycinnamate) | 5,0 % |
| Butylparaben | 0,2 % |
| Germal 115 (Imidazolidinyl urea) | 0,2 % |
| Eau | qsp 100 |
| pH de la formule : 1,3 | |

### Exemple 12 : exemple comparatif

La composition décrite dans l'exemple 1 a fait l'objet d'une étude clinique dans le traitement du vieillissement cutané physiologique en comparaison avec un produit de référence ayant la composition suivante :

Acide salicylique 1 % Excipient qsp 100 % commercialisé sous la marque TURNAROUND ®

30 jurés ont participé à l'étude en aveugle et ont utilisé les produits ci-dessus comparativement pendant 5 jours : chacun des produits était appliqué en quantité égale par demi-visage.

Sur les critères des signes du vieillissement cutané physiologique, les 2 produits ont une action non significativement différente.

Du point de vue confort et tolérance, un seul juré sur 30 a stoppé le test avec la formule de l'exemple 1 alors qu'avec la crème de référence, 7 jurés ont stoppé le test.

Les formules objet de la présente invention ont donc une efficacité prouvée dans le traitement du vieillissement cutané physiologique sans manifester les inconvénients des préparations de l'art antérieur.

## Revendications

1. Utilisation d'au moins un composé présentant au moins une fonction acide sulfonique au moins partiellement non neutralisée dans et pour la fabrication d'une composition cosmétique et/ou dermatologique pour lutter contre le vieillissement intrinsèque.

2. Utilisation selon la revendication 1 dans et pour la fabrication d'une composition cosmétique et/ou dermatologique pour lutter contre les rides et/ou ridules dues au vieillissement intrinsèque.

3. Utilisation selon la revendication 1 dans et pour la fabrication d'une composition cosmétique et/ou dermatologique pour éliminer les cellules mortes de la peau.

4. Utilisation selon l'une des revendications 1 à 3, caractérisée en ce que le dit composé a pour formule générale
RSO₃H (a)
dans laquelle R représente un radical hydrocarboné aliphatique ou aromatique.

5. Utilisation selon la revendication 4, caractérisée en ce que R est un reste hydrocarboné aliphatique choisi parmi le groupe comprenant les radicaux alkyle ayant de 1 à 30 atomes de carbone et alcényle ayant de 2 à 30 atomes de carbone, linéaires ou ramifiés, éventuellement substitués par un ou plusieurs groupements hydroxyle, alcoxy, acyloxy, aryle, cycloalkyle ou polycycloalkyle.

6. Utilisation selon la revendication 4 ou 5, caractérisée en ce que R est le radical CH₃ ou CH₃-(CH₂)₁₁.

7. Utilisation selon la revendication 4 ou 5 caractérisée en ce que le dit composé est l'acide campho-10-sulfonique.

8. Utilisation selon la revendication 4, caractérisée en ce que R est un reste hydrocarboné aromatique ayant de 6 à 10 atomes de carbone éventuellement substitué par un ou plusieurs groupements hydroxyle, alcoxy, alcényloxy, alkyle, acyloxy, aryle, aryloxy, aroyle, aroyloxy, carboxyle, halogèno ou sulfonyle.

9. Utilisation selon la revendication 8, caractérisée en ce que R est le radical C₆H₅

10. Utilisation selon la revendication 8, caractérisée en ce que R est le radical

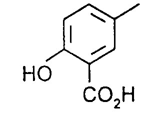

11. Utilisation selon la revendication 4, caractérisée en ce que le dit composé a pour formule générale : 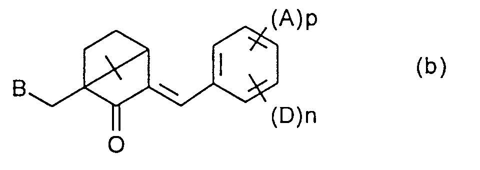 dans laquelle :
B représente H ou SO₃H
0 ≦ p ≦ 1 avec B = SO₃H quand p = 0
0 ≦ n ≦ 4
D représente un ou plusieurs radicaux alkyle, alcényle, alcényloxy ou alcoxy, identiques ou différents, linéaires ou ramifiés contenant de 1 à 18 atomes de carbone, halogéno, hydroxyle
A, de préférence en méta ou en para, représente
soit un radical SO₃H
soit un groupement 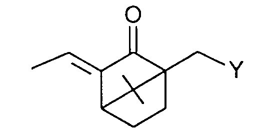 dans lequel Y représente H ou SO₃H
soit un groupement 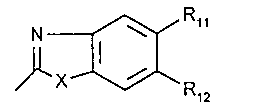 dans lequel
R₁₁ désigne un atome d'hydrogène, un radical alkyle ou alcoxy, linéaire ou ramifié contenant de 1 à 6 atomes de carbone environ ou le radical SO₃H, R₁₁ étant SO₃H lorsque B = H,
R₁₂ désigne un atome d'hydrogène, un radical alkyle ou alcoxy, linéaire ou ramifié, contenant de 1 à 6 atomes de carbone environ,
et X est un atome d'oxygène, de soufre ou un groupement -NR-, R étant un atome d'hydrogène ou un radical alkyle, linéaire ou ramifié, contenant de 1 à 6 atomes de carbone.

12. Utilisation selon la revendication 11, caractérisée en ce que le composé de formule (b) a pour formule : 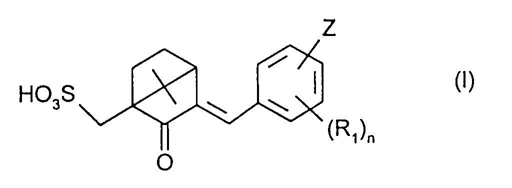 dans laquelle :
- Z, de préférence en position para ou méta, désigne un groupement 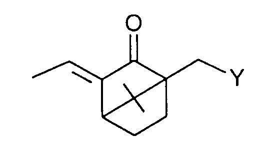 dans lequel Y représente H ou SO₃H
- n est égal à 0 ou est un nombre entier compris entre 1 et 4 (0 ≦n≦ 4)
- R₁, représente un ou plusieurs radicaux alkyle ou alkoxy, identiques ou différents, linéaires ou ramifiés, contenant de 1 à 4 atomes de carbone.

13. Utilisation selon la revendication 12, caractérisée en ce que le composé de formule (I) est l'acide benzène 1,4 [di(3-méthylidènecampho 10-sulfonique)].

14. Utilisation selon la revendication 11, caractérisée en ce que le composé de formule (b) a pour formule : 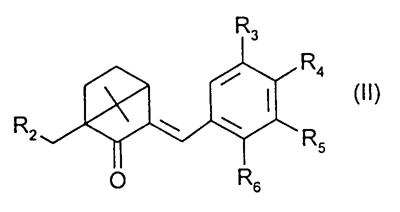 dans laquelle :
- R₂ désigne un atome d'hydrogène ou un radical -SO₃H.
- R₃, R_{4,} R₅ et R₆, identiques ou différents, représentent un groupement hydroxyle, un radical alkyle ayant de 1 à 4 atomes de carbone, linéaire ou ramifié, un radical alcényle ayant de 2 à 4 atomes de carbone, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno, un radical R₃ à R₆ seulement pouvant être un radical SO₃H, au moins un des radicaux R₃ à R₆ étant le radical -SO₃H quand R₂ est un atome d'hydrogène.

15. Utilisation selon la revendication 14, caractérisée en ce que le composé de formule (II) est choisi parmi l'acide 4'-sulfo 3-benzylidènecamphre, l'acide 3-benzylidène campho-10 sulfonique, l'acide 4'-méthyl 3'-sulfo 3-benzylidènecamphre, l'acide 4'-chloro 3'-sulfo 3-benzylidènecamphre, l'acide 4'-méthyl 3-benzylidène campho-10-sulfonique, l'acide 3-(3-t-butyl 2-hydroxy 5-méthyl) benzylidène campho-10-sulfonique, l'acide 3-(3-t-butyl 2-hydroxy 5-méthoxy) benzylidène campho-10-sulfonique, l'acide 3-(3,5-diterbutyl 4-hydroxy) benzylidène campho-10-sulfonique, l'acide 4'-méthoxy 3'-sulfo 3-benzylidènecamphre, l'acide 3-(4,5-méthylènedioxy) benzylidène campho-10-sulfonique, l'acide 3-(4-méthoxy) benzylidène campho-10-sulfonique, l'acide 3-(4,5-diméthoxy) benzylidène campho-10-sulfonique, l'acide 3-(4-n. butoxy) benzylidène campho-10-sulfonique, ou l'acide 3-(4-n.butoxy 5-méthoxy) benzylidène campho-10-sulfonique.

16. Utilisation selon la revendication 11, caractérisée en ce que le composé de formule (b) a pour formule : 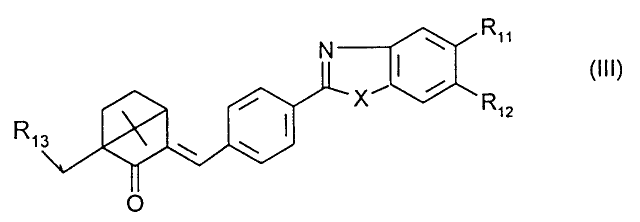 dans laquelle :
- R₁₁ désigne un atome d'hydrogène, un radical alkyle ou alcoxy, linéaire ou ramifié, contenant de 1 à 6 atomes de carbone ou un radical -SO₃H,
- R₁₂ désigne un atome d'hydrogène, un radical alkyle ou alcoxy, linéaire ou ramifié, contenant de 1 à 6 atomes de carbone,
- R₁₃ désigne un atome d'hydrogène ou un radical -SO₃H,
- l'un au moins des radicaux R₁₁ et R₁₃ désignant un radical -SO₃H,
- X est un atome d'oxygène ou de soufre ou un groupement -NR-, R étant un atome d'hydrogène ou un radical alkyle, linéaire ou ramifié, contenant de 1 à 6 atomes de carbone.

17. Utilisation selon la revendication 16, caractérisée en ce que le composé de formule (III) est l'acide 2-[4-(camphométhylidène) phényl] benzimidazole-5-sulfonique.

18. Utilisation selon la revendication 4, caractérisée en ce que ledit composé a pour formule : 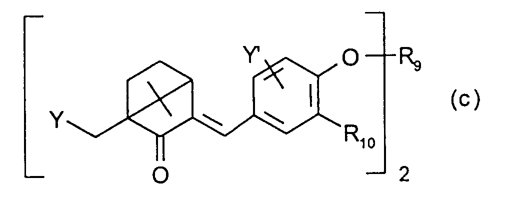 dans laquelle :
- R₉ désigne un radical divalent : -(CH₂)ₘ- ou -CH₂ -CHOH-CH₂- , m étant un nombre entier compris entre 1 et 10 (1 ≦ m ≦ 10),
- R₁₀ désigne un atome d'hydrogène, un radical alcoxy contenant de 1 à 4 atomes de carbone,
- Y et Y' désignent un atome d'hydrogène ou un radical -SO₃H, au moins un de ces radicaux Y ou Y' est différent de l'hydrogène.

19. Utilisation selon la revendication 18, caractérisée en ce que le composé de formule (c) est l'acide éthylène bis [3-(4'-oxy benzylidène) campho-10-sulfonique].

20. Utilisation selon la revendication 4, caractérisée en ce que ledit composé a pour formule : 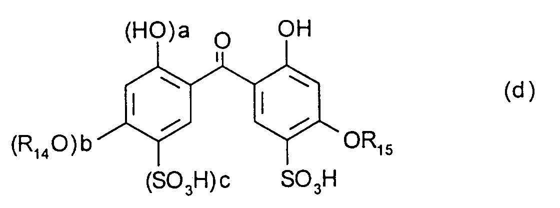 dans laquelle :
- R₁₄ et R₁₅, identiques ou différents, désignent soit un atome d'hydrogène soit un radical alkyle, linéaire ou ramifié, contenant de 1 à 8 atomes de carbone.
- a, b et c, identiques ou différents, sont égaux à 0 ou 1.

21. Utilisation selon la revendication 20, caractérisée en ce que le composé de formule (d) est l'acide 2-hydroxy 4-méthoxybenzophénone 5-sulfonique.

22. Utilisation selon la revendication 4, caractérisée en ce que le dit composé a pour formule générale : 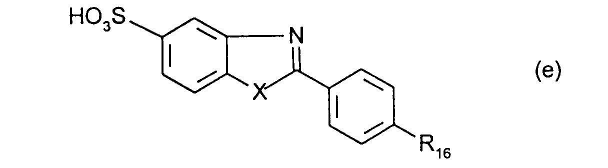 dans laquelle :
- X désigne un atome d'oxygène ou un radical -NH-
- R₁₆ désigne un atome d'hydrogène, un radical alkyle ou alcoxy, linéaire ou ramifié, contenant de 1 à 8 atomes de carbone ou un groupement de formule 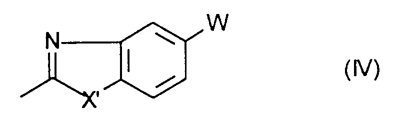 dans laquelle X' représente un atome d'oxygène ou un radical -NH-et W représente H ou SO₃H.

23. Utilisation selon la revendication 22, caractérisée en ce que le composé de formule (e) est l'acide 2-phénylbenzimidazole 5-sulfonique, l'acide benzène 1,4-di(benzimidazol 2 yl 5-sulfonique) ou l'acide benzène 1,4-di(benzoxazol-2 yl -5-sulfonique).

24. Utilisation selon l'une quelconque des revendications précédentes, caractérisée en ce que le dit composé est mis en oeuvre en une quantité comprise entre 0,1 et 10 % en poids de la composition et plus préférentiellement entre 0,1 % et 5 %.

25. Procédé non thérapeutique pour lutter contre le vieillissement cutané intrinsèque, caractérisé en ce qu'il consiste à appliquer sur la peau une composition cosmétique contenant au moins un composé présentant au moins une fonction acide sulfonique au moins partiellement non neutralisée.

## Claims

1. Use of at least one compound having at least one sulphonic acid function which is at least partially non-neutralized in and for the manufacture of a cosmetic and/or dermatological composition for combating intrinsic ageing.

2. Use according to claim 1 in and for the manufacture of a cosmetic and/or dermatological composition for combating wrinkles and/or fine lines due to intrinsic ageing.

3. Use according to claim 1 in and for the manufacture of a cosmetic and/or dermatological composition for removing dead cells from the skin.

4. Use according to one of Claims 1 to 3, characterized in that the said compound has the general formula
RSO₃H (a)
in which R represents an optionally substituted aliphatic or aromatic hydrocarbon radical.

5. Use according to Claim 4, characterized in that R is an aliphatic hydrocarbon residue chosen from the group comprising alkyl radicals having from 1 to 30 carbon atoms and alkenyl radicals having from 2 to 30 carbon atoms, which are linear or branched and are optionally substituted with one or more hydroxyl, alkoxy, acyloxy, aryl, cycloalkyl or polycycloalkyl groups.

6. Use according to Claim 4 or 5, characterized in that R is the radical CH₃ or CH₃-(CH₂)₁₁.

7. Use according to Claim 4 or 5, characterized in that the said compound is camphor-10-sulphonic acid.

8. Use according to Claim 4, characterized in that R is an aromatic hydrocarbon residue having from 6 to 10 carbon atoms, which is optionally substituted with one or more hydroxyl, alkoxy, alkenyloxy, alkyl, acyloxy, aryl, aryloxy, aroyl, aroyloxy, carboxyl, halo or sulphonyl groups.

9. Use according to Claim 8, characterized in that R is the C₆H₅ radical.

10. Use according to Claim 8, characterized in that R is the radical

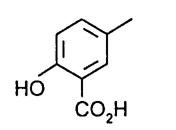

11. Use according to Claim 4, characterized in that the said compound has the general formula: 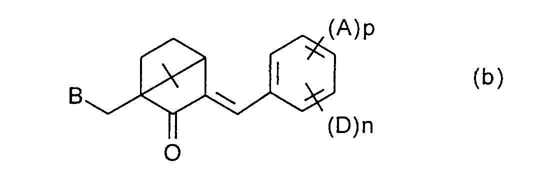 in which:
B represents H or SO₃H
0 ≦ p ≦ 1 with B = SO₃H when p = 0
0 ≦ n ≦ 4
D represents one or more linear or branched alkyl or alkenyl or alkenyloxy or alkoxy radicals or halo or hydroxy radicals which may be identical or different when n ≧ 2, the alkyl, alkenyl, alkenyloxy or alkoxy radicals containing from 1 to 18 carbon atoms.
A, preferably in the meta or para position, represents either an SO₃H radical
or a group 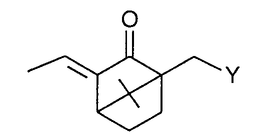 in which Y représents H or SO₃H ;
or a group 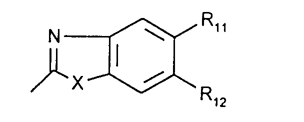 in which
R₁₁ denotes a hydrogen atom, a linear or branched alkyl or alkoxy radical containing from 1 to 6 carbon atoms approximately or the SO₃H radical, R₁₁ being SO₃H when B = H,
R₁₂ denotes a hydrogen atom or a linear or branched alkyl or alkoxy radical containing from 1 to 6 carbon atoms approximately,
and X is an oxygen or sulphur atom or a group -NR-, R being a hydrogen atom or a linear or branched alkyl radical containing from 1 to 6 carbon atoms.

12. Use according to Claim 11, characterized in that the compound of formula (b) has the formula: 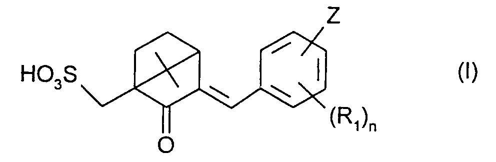 in which:
- Z, preferably in the para or meta position, denotes a group 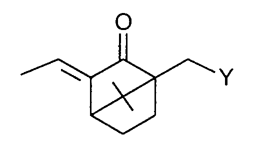 in which Y represents H or SO₃H
- n is equal to 0 or is an integer between 1 and 4 (0 ≦ n ≦ 4)
- R₁ represents one or more linear or branched alkyl or alkoxy radicals, which may be identical or different, containing from 1 to 4 carbon atoms.

13. Use according to Claim 12, characterized in that the compound of formula (I) is benzene-1,4-[di(3-methylidene-10-camphorsulphonic)] acid.

14. Use according to Claim 11, characterized in that the compound of formula (b) has the formula: 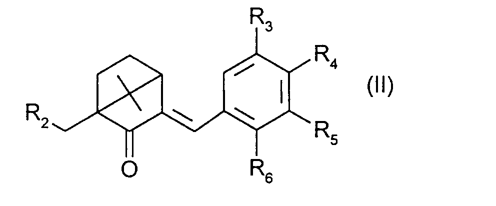 in which:
- R₂ denotes a hydrogen atom or an -SO₃H radical.
- R₃, R₄, R₅ and R₆, which may be identical or different, represent a hydroxyl group, a linear or branched alkyl radical having from 1 to 4 carbon atoms, a linear or branched alkenyl radical having from 2 to 4 carbon atoms, a linear or branched alkoxy radical having from 1 to 4 carbon atoms, a linear or branched alkenyloxy radical having from 2 to 4 carbon atoms, or a halo radical, only one radical R₃ to R₆ possibly being an SO₃H radical, at least one of the radicals R₃ to R₆ being the -SO₃H radical when R₂ is a hydrogen atom.

15. Use according to Claim 14, characterized in that the compound of formula (II) is chosen from 3-benzylidenecamphor-4'-sulphonic acid, 3-benzylidene-10-camphorsulphonic acid, 3-benzylidenecamphor-4'methyl-3'-sulphonic acid, 3-benzylidenecamphor-4'-chloro-3'-sulphonic acid, 4'-methyl-3-benzylidene-10-camphorsulphonic acid, 3-(3-t-butyl-2-hydroxy-5-methyl)benzylidene-10-camphorsulphonic acid, 3-(3-t-butyl-2-hydroxy-5-methoxy)benzylidene-10-camphorsulphonic acid, 3-(3,5-di-ter-butyl-4-hydroxy)benzylidene-10-camphorsulphonic acid, 3-benzylidenecamphor-4'-methoxy-3-sulphonic acid, 3-(4,5-methylenedioxy)benzylidene-10-camphorsulphonic acid, 3-(4-methoxy)benzylidene-10-camphorsulphonic acid, 3-(4,5-dimethoxy)benzylidene-10-camphorsulphonic acid, 3-(4-n-butoxy)benzylidene-10-camphorsulphonic acid or 3-(4-n-butoxy-5-methoxy)benzylidene-10-camphorsulphonic acid.

16. Use according to Claim 11, characterized in that the compound of formula (b) has the formula: 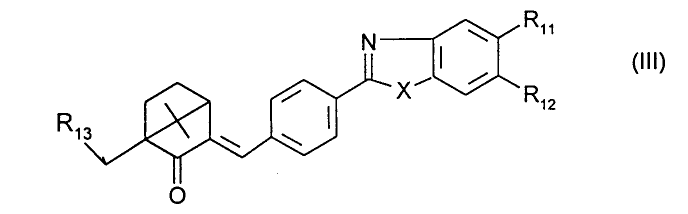 in which:
- R₁₁ denotes a hydrogen atom, a linear or branched alkyl or alkoxy radical containing from 1 to 6 carbon atoms or an -SO₃H radical,
- R₁₂ denotes a hydrogen atom or a linear or branched alkyl or alkoxy radical containing from 1 to 6 carbon atoms,
- R₁₃ denotes a hydrogen atom or an -SO₃H radical,
- at least one of the radicals R₁₁ and R₁₃ denoting an -SO₃H radical,
- X is an oxygen or sulphur atom or a group -NR-, R being a hydrogen atom or a linear or branched alkyl radical containing from 1 to 6 carbon atoms.

17. Use according to Claim 16, characterized in that the compound of formula (III) is 2-[4-(camphomethylidene)phenyl]benzimidazole-5-sulphonic acid.

18. Use according to Claim 4, characterized in that the said compound has the formula: 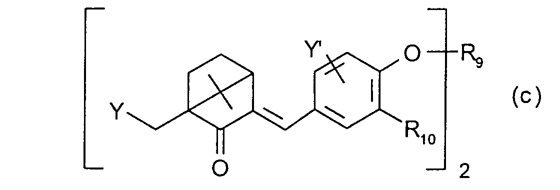 in which:
- R₉ denotes a divalent radical: -(CH₂)ₘ- or -CH₂-CHOH-CH₂-, m being an integer between 1 and 10 (1 ≦ m ≦ 10),
- R₁₀ denotes a hydrogen atom or an alkoxy radical containing from 1 to 4 carbon atoms,
- Y and Y' denote a hydrogen atom or an -SO₃H radical, at least one of these radicals Y or Y' is other than hydrogen.

19. Use according to Claim 18, characterized in that the compound of formula (c) is ethylenebis[3-(4'-oxybenzylidene)-10-camphorsulphoic] acid.

20. Use according to Claim 4, characterized in that the said compound has the formula: 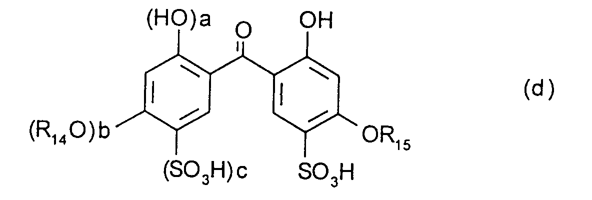 in which:
- R₁₄ and R₁₅, which may be identical or different, denote either a hydrogen atom or a linear or branched alkyl radical containing from 1 to 8 carbon atoms.
- a, b and c, which may be identical or different, are equal to 0 or 1.

21. Use according to Claim 20, characterized in that the compound of formula (d) is 2-hydroxy-4-methoxybenzophenone-5-sulphonic acid.

22. Use according to Claim 4, characterized in that the said compound has the general formula: 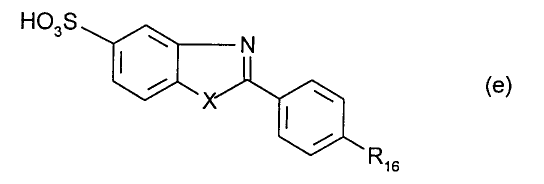 in which:
- X denotes an oxygen atom or an -NH- radical
- R₁₆ denotes a hydrogen atom, a linear or branched alkyl or alkoxy radical containing from 1 to 8 carbon atoms or a group of formula 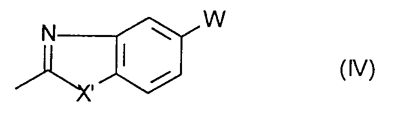 in which X' represents an oxygen atom or an -NH-radical and W represents H or SO₃H.

23. Use according to Claim 22, characterized in that the compound of formula (e) is 2-phenylbenzimidazole-5-sulphonic acid, benzene-1,4-di(benzimidazol-2-yl-5-sulphonic) acid or benzene-1,4-di(benzoxazol-2-yl-5-sulphonic) acid.

24. Use according to any one of the preceding claims, characterized in that the said compound is used in an amount between 0.1 and 10% of the weight of the composition and more preferably between 0.1% and 5%.

25. Non-therapeutic process for combating intrinsic ageing of the skin, characterized in that it consists in applying to the skin a cosmetic and/or dermatological composition containing at least one compound having at least one sulphonic acid function which is at least partially non-neutralized.

## Patentansprüche

1. Verwendung mindestens einer Verbindung, die mindestens eine Sulfonsäuregruppe aufweist, die mindestens teilweise nicht neutralisiert ist, in einer und zur Herstellung einer kosmetischen und/oder dermatologischen Zusammensetzung zur Bekämpfung der physiologischen Hautalterung.

2. Verwendung nach Anspruch 1 in einer und zur Herstellung einer kosmetischen und/oder dermatologischen Zusammensetzung zur Bekämpfung der Falten und/oder Fältchen, die durch die physiologische Hautalterung hervorgerufen werden.

3. Verwendung nach Anspruch 1 in einer und zur Herstellung einer kosmetischen und/oder dermatologischen Zusammensetzung zur Entfernung der abgestorbenen Hautzellen.

4. Verwendung nach einem der Ansprüche 1 bis 3, dadurch gekennzeichnet, daß die obengenannte Verbindung die allgemeine Formel
RSO₃H (a)
aufweist, in der R eine aliphatische oder aromatische Kohlenwasserstoffgruppe darstellt.

5. Verwendung nach Anspruch 4, dadurch gekennzeichnet, daß R eine aliphatische Kohlenwasserstoffgruppe ist, die unter geradkettigen oder verzweigten, ggf. mit einer oder mehreren Hydroxy-, Alkoxy-, Acyloxy-, Aryl-, Cycloalkyl- oder Polycycloalkylgruppen substituierten Alkylgruppen mit 1 bis 30 Kohlenstoffatomen und Alkenylgruppen mit 2 bis 30 Kohlenstoffatomen ausgewählt wird.

6. Verwendung nach Anspruch 4 oder 5, dadurch gekennzeichnet, daß R die Gruppe CH₃ oder CH₃-(CH₂)₁₁- ist.

7. Verwendung nach Anspruch 4 oder 5, dadurch gekennzeichnet, daß es sich bei der Verbindung um Campher-10-sulfonsäure handelt.

8. Verwendung nach Anspruch 4, dadurch gekennzeichnet, daß R ein aromatischer Kohlenwasserstoffrest mit 6 bis 10 Kohlenstoffatomen ist, der ggf. mit einer oder mehreren Hydroxy-, Alkoxy-, Alkenyloxy-, Alkyl-, Acyloxy-, Aryl-, Aryloxy-, Aroyl-, Aroyloxy-, Carboxygruppen, einem oder mehreren Halogenatomen oder einer oder mehreren Sulfonylgruppen substituiert ist.

9. Verwendung nach Anspruch 8, dadurch gekennzeichnet, daß R die Gruppe C₆H₅- ist.

10. Verwendung nach Anspruch 8, dadurch gekennzeichnet, daß R die Gruppe 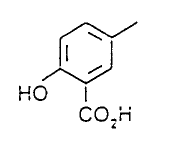 ist.

11. Verwendung nach Anspruch 4, dadurch gekennzeichnet, daß die Verbindung die allgemeine Formel 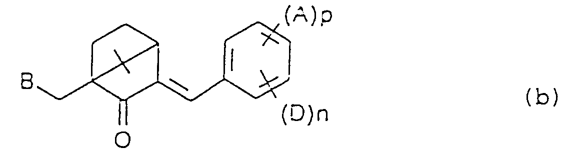 aufweist, in der bedeuten:
- B Wasserstoff oder -SO₃H,
- p 0 oder 1 mit B = -SO₃H, wenn p gleich 0 ist,
- n eine ganze Zahl im Bereich von 0 bis 4, (0 ≦ n ≦ 4)
- D eine oder mehrere geradkettige oder verzweigte Alkyl-, Alkenyl-, Alkenyloxy-, oder Alkoxygruppen, die gleich oder verschieden sind, mit 1 bis 18 Kohlenstoffatomen, ein oder mehrere Halogenatome oder eine oder mehrere Hydroxygruppen,
- die Gruppe A, die sich vorzugsweise in *meta*- oder *para*-Stellung befindet, entweder
eine Gruppe -SO₃H oder
eine Gruppe 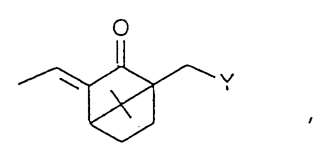 in der Y Wasserstoff oder -SO₃H bedeutet,
oder eine Gruppe 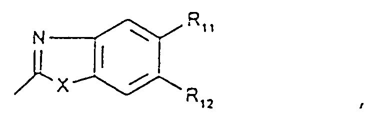 in der bedeuten:
- R₁₁ Wasserstoff, eine geradkettige oder verzweigte Alkyl- oder Alkoxygruppe mit etwa 1 bis 6 Kohlenstoffatomen oder die Gruppe -SO₃H, wobei R₁₁ -SO₃H ist, wenn B Wasserstoff ist,
- R₁₂ Wasserstoff oder eine geradkettige oder verzweigte Alkyl- oder Alkoxygruppe mit etwa 1 bis 6 Kohlenstoffatomen und
- X Sauerstoff, Schwefel oder eine Gruppe -NR-, worin R Wasserstoff oder eine geradkettige oder verzweigte Alkylgruppe mit etwa 1 bis 6 Kohlenstoffatomen ist.

12. Verwendung nach Anspruch 11, dadurch gekennzeichnet, daß die Verbindung der Formel (b) die folgende Formel 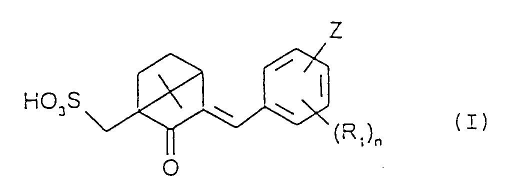 aufweist, in der bedeuten:
- Z eine vorzugsweise in *para*- oder *meta*-Stellung angeordnete Gruppe 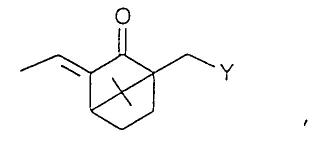 in der Y Wasserstoff oder -SO₃H darstellt,
- n 0 oder eine ganze Zahl von 1 bis 4 (0 ≦ n ≦ 4),
- R₁ eine oder mehrere geradkettige oder verzweigte Alkyl- oder Alkoxygruppen, die gleich oder verschieden sind, mit 1 bis 4 Kohlenstoffatomen.

13. Verwendung nach Anspruch 12, dadurch gekennzeichnet, daß die Verbindung der Formel (I) die 1,4-Benzoldi-(3-methylidencampher-10-sulfonsäure) ist.

14. Verwendung nach Anspruch 11, dadurch gekennzeichnet, daß die Verbindung der Formel (b) die folgende Formel 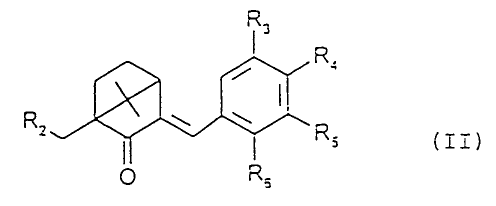 aufweist, in der bedeuten:
- R₂ Wasserstoff oder eine Gruppe -SO₃H,
- R₃, R₄, R₅ und R₆, die gleich oder verschieden sind, eine Hydroxygruppe, eine geradkettige oder verzweigte Alkylgruppe mit 1 bis 4 Kohlenstoffatomen, eine geradkettige oder verzweigte Alkenylgruppe mit 2 bis 4 Kohlenstoffatomen, eine geradkettige oder verzweigte Alkoxygruppe mit 1 bis 4 Kohlenstoffatomen, eine geradkettige oder verzweigte Alkenyloxygruppe mit 2 bis 4 Kohlenstoffatomen, ein Halogen, wobei nur eine der Gruppen R₃ bis R₆ eine Gruppe -SO₃H sein kann, wobei mindestens eine der Gruppen R₃ bis R₆ die Gruppe -SO₃H darstellt, wenn R₂ Wasserstoff ist.

15. Verwendung nach Anspruch 14, dadurch gekennzeichnet, daß die Verbindung der Formel (II) ausgewählt wird unter der Säure 3-(4-Sulfobenzyliden)-campher,
3-Benzylidencampher-10-sulfonsäure,
der Säure 3-(4-Methyl-3-sulfobenzyliden)-campher,
der Säure 3-(4-Chlor-3-sulfobenzyliden)-campher,
3-(4-Methylbenzyliden)-campher-10-sulfonsäure,
3-(3-*tert*.-Butyl-2-hydroxy-5-methylbenzyliden)-campher-10-sulfonsäure,
3-(3-*tert*.-Butyl-2-hydroxy-5-methoxybenzyliden)-campher-10-sulfonsäure,
3-(3,5-Di-*tert*.-butyl-4-hydroxybenzyliden)-campher-10-sulfonsäure,
der Säure 3-(4-Methoxy-3-sulfobenzyliden)-campher,
3-(4,5-Methylendioxybenzyliden)-campher-10-sulfonsäure,
3-(4-Methoxybenzyliden)-campher-10-sulfonsäure,
3-(4,5-Dimethoxybenzyliden)-campher-10-sulfonsäure,
3-(4-n-Butoxybenzyliden)-campher-10-sulfonsäure und
3-(4-n-Butoxy-5-methoxy-benzyliden)-campher-10-sulfonsäure.

16. Verwendung nach Anspruch 11, dadurch gekennzeichnet, daß die Verbindung der Formel (b) die Formel 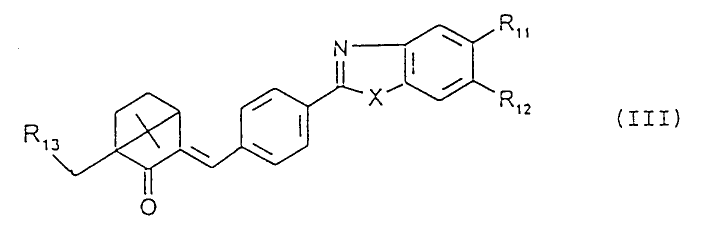 aufweist, in der bedeuten:
- R₁₁ Wasserstoff, eine geradkettige oder verzweigte Alkyl- oder Alkoxygruppe mit 1 bis 6 Kohlenstoffatomen oder eine Gruppe -SO₃H,
- R₁₂ Wasserstoff, eine geradkettige oder verzweigte Alkyl- oder Alkoxygruppe mit 1 bis 6 Kohlenstoffatomen,
- R₁₃ Wasserstoff oder eine Gruppe -SO₃H,
wobei mindestens eine der Gruppen R₁₁ und R₁₃ eine Gruppe
- SO₃H darstellt,
- X Sauerstoff oder Schwefel oder eine Gruppe, -NR-, worin R Wasserstoff oder eine geradkettige oder verzweigte Alkylgruppe mit 1 bis 6 Kohlenstoffatomen darstellt.

17. Verwendung nach Anspruch 16, dadurch gekennzeichnet, daß es sich bei der Verbindung der Formel (III) um 2-[4-(Camphermethyliden)-phenyl]-benzimidazol-5-sulfonsäure handelt.

18. Verwendung nach Anspruch 4, dadurch gekennzeichnet, daß die Verbindung die Formel 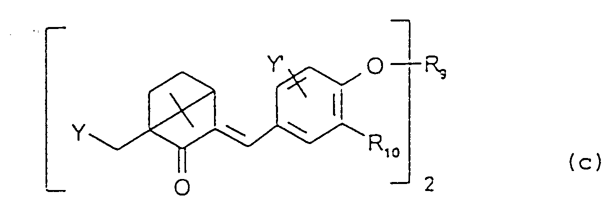 aufweist, in der bedeuten:
- R₉ eine zweiwertige Gruppe -(CH₂)ₘ- oder -CH₂-CHOH-CH₂-, worin m eine ganze Zahl von 1 bis 10 (1 ≦ m ≦ 10) darstellt,
- R₁₀ Wasserstoff oder eine Alkoxygruppe mit 1 bis 4 Kohlenstoffatomen,
- Y und Y' Wasserstoff oder eine Gruppe -SO₃H, wobei mindestens eine der Gruppen Y und Y' verschieden von Wasserstoff ist.

19. Verwendung nach Anspruch 18, dadurch gekennzeichnet, daß es sich bei der Verbindung der Formel (c) um Ethylen-bis[3-(4'-oxybenzyliden)-camper-10-sulfonsäure] handelt.

20. Verwendung nach Anspruch 4, dadurch gekennzeichnet, daß die obengenannte Verbindung die Formel 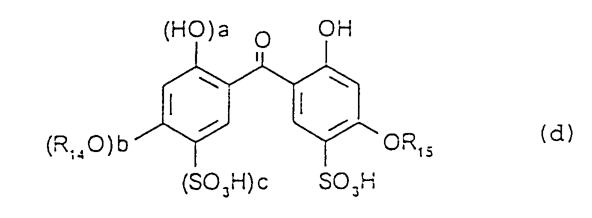 aufweist, in der bedeuten:
- R₁₄ und R₁₅, die gleich oder verschieden sind, Wasserstoff oder eine geradkettige oder verzweigte Alkylgruppe mit 1 bis 8 Kohlenstoffatomen und
- a, b und c, die gleich oder verschieden sind, 0 oder 1.

21. Verwendung nach Anspruch 20, dadurch gekennzeichnet, daß die Verbindung der Formel (d) die 2-Hydroxy-4-methoxybenzophenon-5-sulfonsäure ist.

22. Verwendung nach Anspruch 4, dadurch gekennzeichnet, daß die Verbindung die allgemeine Formel 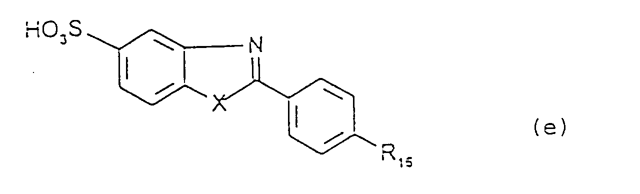 aufweist, in der bedeuten:
- X Sauerstoff oder eine Gruppe -NH- und
- R₁₆ Wasserstoff oder eine geradkettige oder verzweigte Alkyl- oder Alkoxygruppe mit 1 bis 8 Kohlenstoffatomen oder eine Gruppe der Formel 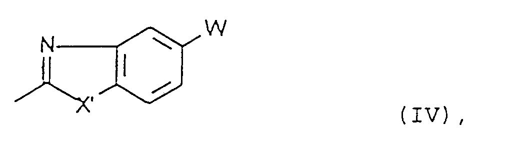 in der X Sauerstoff oder eine Gruppe -NH- und W Wasserstoff oder -SO₃H darstellt.

23. Verwendung nach Anspruch 22, dadurch gekennzeichnet, daß es sich bei der Verbindung der Formel (e) um 2-Phenylbenzimidazol-5-sulfonsäure, 1,4-Benzoldi(benzimidazol-2-yl-5-sulfonsäure) oder 1,4-Benzoldi(benzoxazol-2-yl-5-sulfonsäure) handelt.

24. Verwendung nach einem der vorhergehenden Ansprüche, dadurch gekennzeichnet, daß die obengenannte Verbindung in einer Menge von 0,1 bis 10 Gew.-%, bezogen auf die Zusammensetzung, und vorzugsweise 0,1 bis 5 Gew.-% eingesetzt wird.

25. Nicht-therapeutisches Verfahren zur Bekämpfung der physiologischen Hautalterung, dadurch gekennzeichnet, daß es das Auftragen einer kosmetischen Zusammensetzung auf die Haut umfaßt, die mindestens eine Verbindung enthält, die mindestens eine Sulfonsäuregruppe aufweist, die mindestens teilweise nicht neutralisiert ist.
